# EUROPÄISCHE PATENTSCHRIFT

(11) **EP 1 165 797 B1**
(45) Veröffentlichungstag und Bekanntmachung des Hinweises auf die Patenterteilung: **23.07.2008**
(21) Anmeldenummer: 00909000.2
(22) Anmeldetag: 04.02.2000
(51) Int. Cl.: C12N 15/36, C07K 14/02, C12N 15/62, C07K 19/00, C12N 15/87

(54) **PARTIKEL ZUR GENTHERAPIE**
PARTICLES FOR GENE THERAPY
PARTICULES S'UTILISANT EN THERAPIE GENIQUE

(30) Priorität: 05.02.1999 DE 19904800
(43) Veröffentlichungstag der Anmeldung: 02.01.2002
(73) Patentinhaber: Diaferon GmbH, 80935 München (DE)
(72) Erfinder: HILDT, Eberhard, D-13353 Berlin (DE); HOFSCHNEIDER, Peter, D-80638 München (DE)
(74) Vertreter: Vossius, Volker
(86) Internationale Anmeldenummer: PCT/DE2000/000363
(87) Internationale Veröffentlichungsnummer: WO 2000/046376

(56) Entgegenhaltungen:
- WO-A-93/20221
- WO-A-97/24453
- DE-A- 19 850 718
- DMITRIEV I. ET AL.: "An Adenovirus Vector with Genetically Modified Fibers Demonstrates Expanded Tropism via Utilization of a Coxsackievirus and Adenovirus Receptor-Independent Cell Entry Mechanism" JOURNAL OF VIROLOGY, Bd. 72, Nr. 12, Dezember 1998 (1998-12), Seiten 9706-9713, XP002144482
- OESS S. ET AL.: "Novel cell permeable motif derived from the PreS2-domain of hepatitis-B virus surface antigens" GENE THERAPY, Bd. 7, Nr. 9, Mai 2000 (2000-05), Seiten 750-758, XP000925809
- SCHODEL F ET AL: "Hybrid hepatitis B virus core antigen as a vaccine carrier moiety: I. Presentation of foreign epitopes" JOURNAL OF BIOTECHNOLOGY, Bd. 44, Nr. 1, 26. Januar 1996 (1996-01-26), Seiten 91-96, XP004036853 ISSN: 0168-1656
- DEROSSIT DANIELE ET AL.: THE JOURNAL OF BIOLOGICAL CHEMISTRY, Bd. 269, Nr. 14, Seiten 10444-10459,

### SEQUENZPROTOKOLL

(1) ALLGEMEINE ANGABEN:
   (i) ANMELDER:
      (A) NAME: Dr. Eberhardt Hildt, Klinikum Rechts d. Isar
      (B) STRASSE: Ismaninger Str.
      (C) ORT: Muenchen
      (E) LAND: Deutschland
      (F) POSTLEITZAHL: 81675
   (ii) BEZEICHNUNG DER ERFINDUNG: Partikel zur Gentherapie
   (iii) ANZAHL DER SEQUENZEN: 19
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
   (v) DATEN DER JETZIGEN ANMELDUNG:
      ANMELDENUMMER: DE 199 04 800.2
(2) ANGABEN ZU SEQ ID NO: 1:
   (i) SEQUENZKENNZEICHEN:
      (A) LÄNGE: 347 Aminosäuren
      (B) ART: Aminosäure
      (C) STRANGFORM: Einzelstrang
      (D) TOPOLOGIE: linear
   (ii) ART DES MOLEKÜLS: Peptid
   (xi) SEQUENZBESCHREIBUNG: SEQ ID NO: 1:
   Met Gly Arg Gly Asp Gly Ala Gly Ala Phe Gly Leu Gly Phe Thr Pro
   1               5                   10                  15
   Pro His Gly Gly Leu Leu Gly Trp Ser Pro Gln Ala Gln Gly Ile Leu
               20                  25                  30
   Glu Thr Leu Pro Ala Asn Pro Pro Pro Ala Ser Thr Asn Arg Gln Ser
           35                  40                  45
   Gly Arg Gln Pro Thr Pro Leu Ser Pro Pro Leu Arg Asn Thr His Pro
       50                  55                  60
   Gln Ala Met Gln Trp Asn Ser Thr Thr Phe His Gln Thr Leu Gln Asp
   65                  70                  75                  80
   Pro Arg Val Arg Gly Leu Tyr Phe Pro Ala Gly Gly Ser Ser Ser Gly
                   85                  90                  95
   Thr Val Asn Pro Val Pro Thr Thr Val Ser Pro Ile Ser Ser Ile Phe
               100                 105                 110
   Ser Arg Ile Gly Asp Pro Ala Leu Asn Met Glu Asn Ile Thr Ser Gly
           115                 120                 125
   Phe Leu Gly Pro Leu Leu Val Leu Gln Ala Gly Phe Phe Leu Leu Thr
       130                 135                 140
   Arg Ile Leu Thr Ile Pro Gln Ser Leu Asp Ser Trp Trp Thr Ser Leu
   145                 150                 155                 160
   Asn Phe Leu Gly Gly Thr Thr Val Cys Leu Gly Gln Asn Ser Gln Ser
                   165                 170                 175
   Pro Thr Ser Asn His Ser Pro Thr Ser Cys Pro Pro Thr Cys Pro Gly
               180                 185                 190
   Tyr Arg Trp Met Cys Leu Arg Arg Phe Ile Ile Phe Leu Phe Ile Leu
           195                 200                 205
   Leu Leu Cys Leu Ile Phe Leu Leu Val Leu Leu Asp Tyr Gln Gly Met
       210                 215                 220
   Leu Pro Val Cys Pro Leu Ile Pro Gly Ser Ser Thr Thr Ser Thr Gly
   225                 230                 235                 240
   Pro Cys Arg Thr Cys Thr Thr Pro Ala Gln Gly Thr Ser Met Tyr Pro
                   245                 250                 255
   Ser Cys Cys Cys Thr Lys Pro Ser Asp Gly Asn Cys Thr Cys Ile Pro
               260                 265                 270
   Ile Pro Ser Ser Trp Ala Phe Gly Lys Phe Leu Trp Glu Trp Ala Ser
           275                 280                 285
   Ala Arg Phe Ser Trp Leu Ser Leu Leu Val Pro Phe Val Gln Trp Phe
       290                 295                 300
   Val Gly Leu Ser Pro Thr Val Trp Leu Ser Val Ile Trp Met Met Trp
   305                 310                 315                 320
   Tyr Trp Gly Pro Ser Leu Tyr Ser Ile Leu Ser Pro Phe Leu Pro Leu
                   325                 330                 335
   Leu Pro Ile Phe Phe Cys Leu Trp Val Tyr Ile
               340                 345
(2) ANGABEN ZU SEQ ID NO: 2:
   (i) SEQUENZKENNZEICHEN:
      (A) LÄNGE: 215 Aminosäuren
      (B) ART: Aminosäure
      (C) STRANGFORM: Einzelstrang
      (D) TOPOLOGIE: linear
   (ii) ART DES MOLEKÜLS: Peptid
   (xi) SEQUENZBESCHREIBUNG: SEQ ID NO: 2:
   Met Pro Leu Ser Ser Ile Phe Ser Arg Ile Gly Asp Pro Thr Val Gln
   1               5                   10                  15
   Ala Ser Lys Leu Cys Leu Gly Trp Leu Trp Gly Met Asp Ile Asp Pro
               20                  25                  30
   Tyr Lys Glu Phe Gly Ala Thr Val Glu Leu Leu Ser Phe Leu Pro Ser
           35                  40                  45
   Asp Phe Phe Pro Ser Val Arg Asp Leu Leu Asp Thr Ala Ser Ala Leu
       50                  55                  60
   Tyr Arg Glu Ala Leu Glu Ser Pro Glu His Cys Ser Pro His His Thr
   65                  70                  75                  80
   Ala Leu Arg Gln Ala Ile Leu Cys Trp Gly Glu Leu Met Thr Leu Ala
                   85                  90                  95
   Thr Trp Val Gly Val Asn Leu Glu Asp Pro Glu Phe Arg Gly Asp Ala
               100                 105                 110
   Ser Arg Asp Leu Val Val Ser Tyr Val Asn Thr Asn Met Gly Leu Lys
           115                 120                 125
   Phe Arg Gln Leu Leu Trp Phe His Ile Ser Cys Leu Thr Phe Gly Arg
       130                 135                 140
   Glu Thr Val Ile Glu Tyr Leu Val Ser Phe Gly Val Trp Ile Arg Thr
   145                 150                 155                 160
   Pro Pro Ala Tyr Arg Pro Pro Asn Ala Pro Ile Leu Ser Thr Leu Pro
                   165                 170                 175
   Glu Thr Thr Val Val Arg Arg Arg Gly Arg Ser Pro Arg Arg Arg Thr
               180                 185                 190
   Pro Ser Pro Arg Arg Arg Arg Ser Gln Ser Pro Arg Arg Arg Arg Ser
           195                 200                 205
   Gln Ser Arg Glu Pro Gln Cys
       210                 215
(2) ANGABEN ZU SEQ ID NO: 3:
   (i) SEQUENZKENNZEICHEN:
      (A) LÄNGE: 663 Basenpaare
      (B) ART: Nucleotid
      (C) STRANGFORM: Einzelstrang
      (D) TOPOLOGIE: linear
   (ii) ART DES MOLEKÜLS: cDNA
   (xi) SEQUENZBESCHREIBUNG: SEQ ID NO: 3:
   ATGCCCATAT CGTCAATCTT CTCGAGGATT GGGGACCCTG GATCCACTAC TGTTCAAGCC     60
   TCCAAGCTGT GCCTTGGGTG GCTTTGGGGC ATGGACATCG ACCCTTATAA AGAATTTGGA    120
   GCTACTGTGG AGTTACTCTC GTTTTTGCCT TCTGACTTCT TTCCTTCAGT ACGAGATCTT    180
   CTAGATACCG CCTCAGCTCT GTATCGGGAA GCCTTAGAGT CTCCTGAGCA TTGTTCACCT    240
   CACCATACTG CACTCAGGCA AGCAATTCTT TGCTGGGGGG AACTAATGAC TCTAGCTACC    300
   TGGGTGGGTG TTAATTTGGA AGATCCAGAA TTCCGAGGCG ACGCGTCTAG AGACCTAGTA    360
   GTCAGTTATG TCAACACTAA TATGGGCCTA AAGTTCAGGC AACTCTTGTG GTTTCACATT    420
   TCTTGTCTCA CTTTTGGAAG AGAAACCGTT ATAGAGTATT TGGTGTCTTT CGGAGTGTGG    480
   ATTCGCACTC CTCCAGCTTA TAGACCACCA AATGCCCCTA TCCTATCAAC ACTTCCGGAA    540
   ACTACTGTTG TTAGACGACG AGGCAGGTCC CCTAGAAGAA GAACTCCCTC GCCTCGCAGA    600
   CGAAGGTCTC AATCGCCGCG TCGCAGAAGA TCTCAATCTC GGGAACCTCA ATGTTAGTAT    660
   TCC                                                                  663
(2) ANGABEN ZU SEQ ID NO: 4:
   (i) SEQUENZKENNZEICHEN:
      (A) LÄNGE: 1047 Basenpaare
      (B) ART: Nucleotid
      (C) STRANGFORM: Einzelstrang
      (D) TOPOLOGIE: linear
   (ii) ART DES MOLEKÜLS: cDNA
   (xi) SEQUENZBESCHREIBUNG: SEQ ID NO: 4:
   ATGGGCCGTG GCGAAGGAGC TGGAGCATTC GGGCTGGGTT TCACCCCACC GCACGGAGGC     60
   CTTTTGGGGT GGAGCCCTCA GGCTCAGGGC ATACTACAAA CTTTGCCAGC AAATCCGCCT    120
   CCTGCCTCCA CCAATCGCCA GACAGGAAGG CAGCCTACCC CGCTGTCTCC ACCTTTGAGA    180
   AACACTCATC CTCAGGCCAT GCAGTGGAAT TCCACAACCT TTCACCAAAC TCTGCAAGAT    240
   CCCAGAGTGA GAGGCCTGTA TTTCCCTGCT GGTGGCTCCA GTTCAGGAGC AGTAAACCCT    300
   GTTCCGACTA CTGCCTCTCC CTTATCGTCA ATCTTCTCGA GGATTGGGGA CCCTGCGCTG    360
   AACATGGAGA ACATCACATC AGGATTCCTA GGACCCCTTC TCGTGTTACA GGCGGGGTTT    420
   TTCTTGTTGA CAAGAATCCT CACAATACCG CAGAGTCTAG ACTCGTGGTG GACTTCTCTC    480
   AATTTTCTAG GGGGAACTAC CGTGTGTCTT GGCCAAAATT CGCAGTCCCC AACCTCCAAT    540
   CACTCACCAA CCTCCTGTCC TCCAACTTGT CCTGGTTATC GCTGGATGTG TCTGCGGCGT    600
   TTTATCATCT TCCTCTTCAT CCTGCTGCTA TGCCTCATCT TCTTGTTGGT TCTTCTGGAC    660
   TATCAAGGTA TGTTGCCCGT TTGTCCTCTA ATTCCAGGAT CCTCAACCAC CAGCACGGGA    720
   CCATGCCGAA CCTGCATGAC TACTGCTCAA GGAACCTCTA TGTATCCCTC CTGTTGCTGT    780
   ACCAAACCTT CGGACGGAAA TTGCACCTGT ATTCCCATCC CATCATCCTG GGCTTTCGGA    840
   AAATTCCTAT GGGAGTGGGC CTCAGCCCGT TTCTCCTGGC TCAGTTTACT AGTGCCATTT    900
   GTTCAGTGGT TCGTAGGGCT TTCCCCCACT GTTTGGCTTT CAGTTATATG GATGATGTGG    960
   TATTGGGGGC CAAGTCTGTA CAGCATCTTG AGTCCCTTTT TACCGCTGTT ACCAATTTTC   1020
   TTTTGTCTTT GGGTATACAT TTAAACC                                       1047
(2) ANGABEN ZU SEQ ID NO: 5:
   (i) SEQUENZKENNZEICHEN:
      (A) LÄNGE: 35 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Primer"
   (xi) SEQUENZBESCHREIBUNG: SEQ ID NO: 5:
      CCATATTCTT GGGAACAAGA TATCCAGCAC GGGGC 35
(2) ANGABEN ZU SEQ ID NO: 6:
   (i) SEQUENZKENNZEICHEN:
      (A) LÄNGE: 33 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Primer"
   (xi) SEQUENZBESCHREIBUNG: SEQ ID NO: 6:
      GGATTGCTGG TGGAAGATAT CTGCCCCGTG CTG 33
(2) ANGABEN ZU SEQ ID NO: 7:
   (i) SEQUENZKENNZEICHEN:
      (A) LÄNGE: 33 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Primer"
   (xi) SEQUENZBESCHREIBUNG: SEQ ID NO: 7:
      CAGCACGGGG CAGATATCTT CCACCAGCAA TCC 33
(2) ANGABEN ZU SEQ ID NO: 8:
   (i) SEQUENZKENNZEICHEN:
      (A) LÄNGE: 38 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Primer"
   (xi) SEQUENZBESCHREIBUNG: SEQ ID NO: 8:
      GCCCCGTGCT GGATATCATC TTGTTCCCAA GAATATGG 38
(2) ANGABEN ZU SEQ ID NO: 9:
   (i) SEQUENZKENNZEICHEN:
      (A) LÄNGE: 36 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Primer"
   (xi) SEQUENZBESCHREIBUNG: SEQ ID NO: 9:
      AAAAGATCTG GCCGTGGCGA AGGAGCTGGA GCATTC 36
(2) ANGABEN ZU SEQ ID NO: 10:
   (i) SEQUENZKENNZEICHEN:
      (A) LÄNGE: 30 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Primer"
   (xi) SEQUENZBESCHREIBUNG: SEQ ID NO: 10:
      AAAAGATCTG GTTTAAATGT ATACCCAAAG 30
(2) ANGABEN ZU SEQ ID NO: 11:
   (i) SEQUENZKENNZEICHEN:
      (A) LÄNGE: 33 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Primer"
   (xi) SEQUENZBESCHREIBUNG: SEQ ID NO: 11:
      CCCGATATCA TGTCATCTCT TGTTCATGTC CTA 33
(2) ANGABEN ZU SEQ ID NO: 12:
   (i) SEQUENZKENNZEICHEN:
      (A) LÄNGE: 30 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Primer"
   (xi) SEQUENZBESCHREIBUNG: SEQ ID NO: 12:
      GGGGATATCG GTCGATGTCC ATGCCCCAAA 30
(2) ANGABEN ZU SEQ ID NO: 13:
   (i) SEQUENZKENNZEICHEN:
      (A) LÄNGE: 36 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Primer"
   (xi) SEQUENZBESCHREIBUNG: SEQ ID NO: 13:
      GGGGGATCCC GATGTACGGG CCAGATATAC GCGTTG 36
(2) ANGABEN ZU SEQ ID NO: 14:
   (i) SEQUENZKENNZEICHEN:
      (A) LÄNGE: 27 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Primer"
   (xi) SEQUENZBESCHREIBUNG: SEQ ID NO: 14:
      GGGGGATCCG CGGCCGCTTT ACTTGTA 27
(2) ANGABEN ZU SEQ ID NO: 15:
   (i) SEQUENZKENNZEICHEN:
      (A) LÄNGE: 57 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Primer"
   (xi) SEQUENZBESCHREIBUNG: SEQ ID NO: 15:
      NNNAGATCTA TGCCCATATC GTCAATCTTC TCGAGGATTG GGGACCCTGG ATCCNNN 57
(2) ANGABEN ZU SEQ ID NO: 16:
   (i) SEQUENZKENNZEICHEN:
      (A) LÄNGE: 30 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Primer"
   (xi) SEQUENZBESCHREIBUNG: SEQ ID NO: 16:
      NNNGGATCCA CTGTTCAAGC CTCCAAGCTG 30
(2) ANGABEN ZU SEQ ID NO: 17:
   (i) SEQUENZKENNZEICHEN:
      (A) LÄNGE: 36 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Primer"
   (xi) SEQUENZBESCHREIBUNG: SEQ ID NO: 17:
      NNNGAATTCT GGATCTTCCA AATTAACACC CACCCA 36
(2) ANGABEN ZU SEQ ID NO: 18:
   (i) SEQUENZKENNZEICHEN:
      (A) LÄNGE: 39 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Primer"
   (xi) SEQUENZBESCHREIBUNG: SEQ ID NO: 18:
      NNNGAATTCC GAGGCGACGC GTCTAGAGAC CTAGTAGTC 39
(2) ANGABEN ZU SEQ ID NO: 19:
   (i) SEQUENZKENNZEICHEN:
      (A) LÄNGE: 30 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Primer"
   (xi) SEQUENZBESCHREIBUNG: SEQ ID NO: 19:
      NNNAAGCTTT CCCCACCTTA TGAGTCCAAG 30

## Beschreibung

Die vorliegende Erfindung betrifft Nukleinsäure enthaltende Partikel, die gezielt an Zellen binden und in diese ihre Nukleinsäure einführen können. Ferner betrifft die Erfindung Verfahren zur Herstellung solcher Partikel und hierfür geeignete Mittel sowie die Verwendung der Partikel zur Herstellung einer pharmazeutischen Zusammensetzung zur Gentherapie.

Für eine Gentherapie ist es wichtig ein Gentransfersystem zu haben, das spezifisch ist, d.h. mit dem gewünschte Zellen erreicht und in diese Gene eingeführt werden können. Bei Leberzellen ist dies prinzipiell mit einem modifizierten Hepatitis B-Virus (HBV) als Vektor möglich, da HBV leberzellspezifisch ist. Für andere Zellen, z.B. Fibroblasten, existiert jedoch kein Gentransfersystem, das befriedigende Ergebnisse liefert.

Der vorliegenden Erfindung liegt somit die Aufgabe zugrunde, ein Gentransfersystem bereitzustellen, das spezifisch ist, d.h. mit dem gewünschte Zellen erreicht und in diese Gene eingeführt werden können.

Erfindungsgemäß wird dies durch die Gegenstände in den Patentansprüchen erreicht.

Die vorliegende Erfindung beruht auf den Erkenntnissen des Anmelders, daß Nukleinsäure enthaltende Partikel, die ein Fusionsprotein aufweisen, welches ein Virus-Protein, ein Zellpermeabilität-vermittelndes Peptid, insbesondere ein solches der deutschen Patentanmeldung 198 50 718.6, und eine heterologe zellspezifische Bindungsstelle umfaßt, an entsprechende Zellen binden und in diese ihre Nukleinsäure einführen können. Beispielsweise hat er Nukleinsäure enthaltende HBV-Partikel hergestellt, die an Fibroblasten binden und in diese ihre Nukleinsäure einführen. Hierzu hat er die Hepatocyten-Bindungsstelle, die in der Region PreS1, insbesondere zwischen den Aminosäuren 21-47, des großen Oberflächenproteins von HBV (LHBs) vorliegt, gegen die α5β1-Integrin-Bindungsstelle von Fibronektin ausgetauscht, wobei das Zellpermeabilität-vermittelnde Peptid, das in der Region PreS2 von LHBs vorliegt, erhalten blieb. Ferner hat er Partikel mit Fibroblasten-Spezifität hergestellt, indem er das Core-Protein von HBV (HBcAg) mit der α5β1-Integrin-Bindungsstelle von Fibronektin und dem vorstehenden Zellpermeabilitätvermittelnden Peptid verbunden hat. Desweiteren hat er erkannt, daß die in den Partikeln enthaltene Nukleinsäure in den Zellen exprimiert wird.

Erfindungsgemäß werden die Erkenntnisse des Anmelders genutzt, Partikel bereitzustellen, umfassend:
(a) eine Proteinhülle mit einem Fusionsprotein, das ein Virus-Protein, ein Zellpermeabilität-vermittelndes Peptid und eine heterologe zellspezifische Bindungsstelle umfaßt, und
(b) eine in der Proteinhülle vorliegende Nukleinsäure, die Sequenzen für ein Virus-spezifisches Verpackungssignal und ein Struktur-Gen aufweist,
wobei das Zellpermeabilität-vermittelnde Peptid die Aminosäuresequenz PLSSIFSRIGDP oder eine Aminosäuresequenz, die zu der Aminosäuresequenz PLSSIFSRIGDP in einer Aminosäure unterschiedlich ist, umfasst, wobei die Aminosäuresequenz, die zu der Aminosäuresequenz PLSSIFSRIGDP in einer Aminosäure unterschiedlich ist, von einer Nukleinsäuresequenz kodiert wird, die mit der Nukleinsäuresequenz CCC CTA TCG TCA ATC TTC TCG AGG ATT GGG GAC CCT hybridisiert.

Der Ausdruck "Zellpermeabilität-vermittelndes Peptid" umfaßt Peptide, die Substanzen, insbesondere Proteinen, eine Zellpermeabilität vermitteln können, insbesondere ein Peptid, das die folgende Aminosäure-(DNA)-Sequenz umfaßt:

Der Ausdruck "zellspezifische Bindungsstelle" umfaßt jegliche Bindungsstellen von Proteinen und sonstigen kleinen Molekülen, über welche die Proteine bzw. die Moleküle an Zellen binden können. Beispiele solcher Bindungsstellen finden sich in Cytokinen und Wachstumsfaktoren. Ferner finden sie sich .in Liganden von Hormon-, Neurotransmitter-, Blutzellenoberflächen- und Integrin-Rezeptoren. Eine bevorzugte Bindungsstelle ist die α5β1-Integrin-Bindungsstelle von Fibronektin. Diese wird nachstehend mit RGD bezeichnet und umfaßt die Aminosäuren Arginin, Glycin und Aspartat.

Der Ausdruck "Virus" umfaßt DNA- und RNA-Viren, insbesondere Adenoviren, Adeno-assoziierte Viren, Vacciniaviren, Baculoviren, Hepatitis C-Viren, Hepatitis A-Viren, Influenzaviren und Hepadna-Viren. Beispiele letzterer sind HBV, WHV ("woodchuck hepatitis virus"), GSHV ("ground squirrel hepatitis virus"), RBSHV ("red-bellied squirrel hepatitis virus"), DHV ("Pekin duck hepatitis virus") und HHV ("heron hepatitis virus"), wobei HBV bevorzugt ist.

Der Ausdruck "Virus-Protein" betrifft jegliches Protein eines vorstehenden Virus, das als ganzes oder teilweise in einem Fusionsprotein zusammen mit einem Zellpermeabilität-vermittelnden Peptid und einer heterologen zellspezifischen Bindungsstelle in Form eines weiteren Peptids vorliegen kann. Das Protein kann auch bereits das Zellpermeabilität-vermittelnde Peptid enthalten. Ein Beispiel für ein solches Protein ist LHBs. Dieses wird wie andere Oberflächen-Proteine und Core-Proteine, z.B. HBcAg, bevorzugt. Der Ausdruck "heterolog" weist darauf hin, daß das Protein von Natur aus nicht die vorstehende zellspezifische Bindungsstelle aufweist. Günstig kann es sein, wenn die homologe, d.h. von Natur aus vorliegende Bindungsstelle des Proteins ausgeschaltet ist. Besonders günstig kann es sein, wenn die homologe durch die heterologe Bindungsstelle ersetzt ist.

Der Ausdruck "Nukleinsäure" umfaßt RNA und DNA, wobei beide einzelsträngig und/oder doppelsträngig sein können.

Der Ausdruck "Virus-spezifisches Verpackungssignal" weist auf eine Signalsequenz in vorstehender Nukleinsäure hin, mittels dieser die Nukleinsäure in die Proteinhülle eines Partikels verpackt wird. Die Signalsequenz ist spezifisch für ein vorstehendes Virus. Eine bevorzugte Signalsequenz ist jene von HBV. Diese findet sich auf der HBV-DNA und wird in der Literatur mit Epsilon bezeichnet.

Der Ausdruck "Struktur-Gen" umfaßt Gene, die für Polypeptide (Proteine) kodieren. Beispiele von Polypeptiden sind Tumornekrosefaktor, Interferone, Interleukine, Lymphokine, Wachstumsfaktoren, Plasmaproteine, z.B. Gerinnungsfaktoren und Stoffwechselenzyme, und Rezeptoren. Insbesondere können die Polypeptide solche sein, welche die Immunogenität von Zellen steigern können. Dies können Polypeptide sein, die Tumorzellen fehlen, z.B. Cytokine, wie IL-2 und GM-CSF, und kostimulatorische Moleküle, wie B7-1, Tumor-assoziierte Antigene, z.B. MAGE1, Tyrosinasen und virale Polypeptide, z.B. E7 von humanem Papillomvirus und EBNA-3-Polypeptid von Epstein-Barr-Virus. Ferner können die Polypeptide Adapter-Polypeptide, Oligomerisierungsmotive eines Polypeptids, Polypeptidfragmente von Virus-Hüllpolypeptiden und Hormone sein. Ferner umfaßt der Ausdruck "Struktur-Gen" Antisense-Oligonukleotide, Peptid-Nukleinsäuren, Consensus-Sequenzen für Transkriptionsfaktoren und Ribozyme.

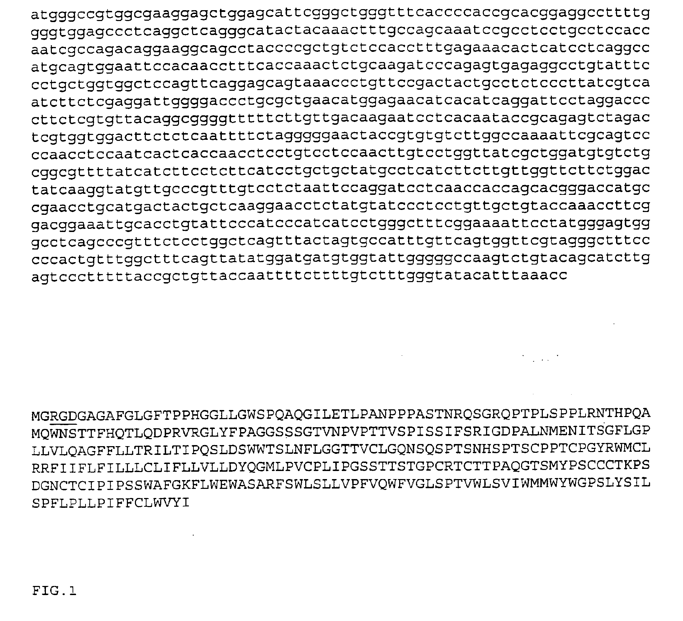

Erfindungsgemäß werden Partikel bevorzugt, die ein Fusionsprotein enthalten, das ein LHBs oder Fragmente davon und eine heterologe Bindungsstelle, insbesondere RGD, umfaßt. Günstig ist es, wenn die heterologe Bindungsstelle, insbesondere RGD, anstelle der homologen Bindungsstelle vorliegt. Besonders bevorzugt ist es, wenn das Fusionsprotein die Aminosäuresequenz von Fig. 1 oder eine hiervon durch eine oder mehrere Aminosäuren unterschiedliche Aminosäuresequenz aufweist.

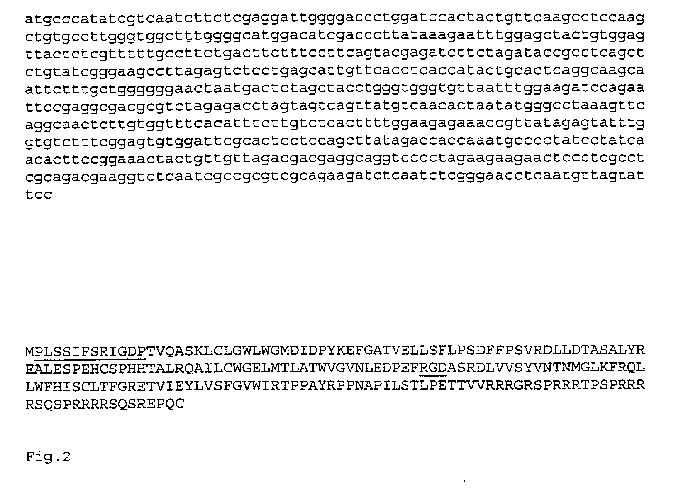

Des weiteren werden Partikel bevorzugt, die ein Fusionsprotein enthalten, das ein HBcAg, ein Zellpermeabilität-vermittelndes Peptid wie vorstehend beschrieben, insbesondere mit der vorstehend angegebenen Aminosäuresequenz, und eine heterologe Bindungsstelle, insbesondere RGD, umfaßt. Besonders bevorzugt ist es, wenn das Fusionsprotein die Aminosäuresequenz von Fig. 2 oder eine hiervon durch eine oder mehrere Aminosäuren unterschiedliche Aminosäuresequenz aufweist.

Der Ausdruck "eine durch eine oder mehrere Aminosäuren unterschiedliche Aminosäuresequenz" weist darauf hin, daß diese Aminosäuresequenz ein Fusionsprotein kennzeichnet, das vergleichbare Elemente und Funktionen wie das Fusionsprotein von Fig. 1 oder Fig. 2 aufweist, sich allerdings bis zu 20 %, vorzugsweise 10 %, von der Aminosäuresequenz von Fig. 1 oder 2 unterscheidet.

Ein erfindungsgemäßes Partikel kann durch übliche Verfahren hergestellt werden. Enthält das Partikel z.B. ein Fusionsprotein, das ein LHBs umfaßt, in dem die homologe durch eine heterologe Bindungsstelle, insbesondere RGD, ersetzt ist, so ist ein Verfahren günstig, das folgende Verfahrensschritte aufweist:
(a) Co-Transfektion von Zellen, die für ein Heptatitis B-Virus-Genom kodieren, wobei diese kein LHBs exprimieren, mit einem ersten Expressionsvektor, der für ein Fusionsprotein kodiert, das ein LHBs umfaßt, in dem die homologe durch eine heterologe Bindungsstelle, insbesondere RGD, ersetzt ist, und mit einem zweiten Expressionsvektor, der ein Virus-spezifisches Verpackungssignal und ein Struktur-Gen aufweist, und
(b) Isolierung und Reinigung des Partikels.

Enthält das Partikel ein Fusionsprotein, das ein HBcAG, ein Zellpermeabilität-vermittelndes Peptid wie vorstehend beschrieben, insbesondere jenes mit vorstehender Aminosäuresequenz, und eine heterologe Bindungsstelle, insbesondere RGD, umfaßt, so ist ein Verfahren günstig, das folgende Verfahrensschritte umfaßt:
(a) Co-Transfektion von Zellen, die für eine HBV-polymerase kodieren, mit einem ersten Expressionsvektor, der für ein Fusionsprotein kodiert, das ein HBcAg, ein Zellpermeabilität-vermittelndes Peptid wie vorstehend beschrieben, insbesondere jenes mit vorstehender Aminosäuresequenz, und eine heterologe Bindungsstelle, insbesondere RGD, umfaßt, und mit einem zweiten Expressionsvektor, der ein Virus-spezifisches Verpackungssignal und ein Struktur-Gen aufweist, und
(b) Isolierung und Reinigung des Partikels.

Hinsichtlich der Ausdrücke "Expressionsvektor", "Zellen", und "Isolierung und Reinigung" wird auf nachstehende Ausführungen, insbesondere in den Beispielen, verwiesen. Bezüglich der anderen Ausdrücke wird auf vorstehende Ausführungen verwiesen.

Ein weiterer Gegenstand der vorliegenden erfindung ist ein Fusionsprotein, das ein Virus-Protein, ein Zellpermeabilität-vermittelndes Peptid und eine heterologe zellspezifische Bindungsstelle, insbesondere RGD, umfaßt, wobei das Zellpermeabilität-vermittelnde Peptid die Aminosäuresequenz PLSSIFSRIGDP oder eine Aminosäuresequenz, die zu der Aminosäuresequenz PLSSIFSRIGDP in einer Aminosäure unterschiedlich ist, umfasst, wobei die Aminosäuresequenz, die zu der Aminosäuresequenz PLSSIFSRIGDP in einer Aminosäure unterschiedlich ist, von einer Nukleinsäuresequenz kodiert wird, die mit der Nukleinsäuresequenz CCC CTA TCG TCA ATC TTC TCG AGG ATT GGG GAC CCT hybridisiert. Vorzugsweise ist das Virus-Protein ein LHBs oder ein HBcAg. Vorzugsweise umfaßt das Fusionsprotein die Aminosäuresequenz von Fig. 1 oder 2 oder eine hiervon durch ein oder mehrere Aminosäuren unterschiedliche Aminosäuresequenz. Hinsichtlich des Ausdrucks "eine durch eine oder mehrere Aminosäuren unterschiedliche Aminosäuresequenz" wird auf vorstehende Ausführungen verwiesen.
Ein weiterer Gegenstand der vorliegenden Erfindung ist eine DNA, die für ein vorstehendes Fusionsprotein kodiert. Bevorzugt ist eine DNA, die folgendes umfaßt:
(a) Die DNA von Fig. 1 oder 2 oder eine hiervon durch ein oder mehrere Basenpaare unterschiedliche DNA, oder
(b) eine mit der DNA von (a) über den degenerierten genetischen Code verwandte DNA.

Der Ausdruck "eine durch ein oder mehrere Basenpaare unterschiedliche DNA" weist darauf hin, daß diese DNA für ein Fusionsprotein kodiert, das vergleichbare Elemente und Funktionen wie das Fusionsprotein von Fig. 1 oder 2 aufweist, sich allerdings von der Basensequenz von Fig. 1 oder 2 derart unterscheidet, daß in der Aminosäuresequenz ein Unterschied von maximal 20 %, vorzugsweise 10 %, vorliegt.

Eine erfindungsgemäße DNA kann als solche oder in einem Vektor vorliegen. Insbesondere kann eine erfindungsgemäße DNA in einem Expressionsvektor vorliegen. Beispiele solcher sind dem Fachmann bekannt. Im Falle eines Expressionsvektors für E. coli sind dies z.B. pGEMEX, pUC-Derivate, pGEX-2T, pET3b und pQE-8. Für die Expression in Hefe sind z.B. pY100 und Ycpad1 zu nennen, während für die Expression in tierischen Zellen z.B. pKCR, pEFBOS, cDM8, pCEV4, pCDNA3, pKSV10, pRCMV und pRK5 anzugeben sind. Für die Expression in Insektenzellen eignet sich besonders der Bacculovirus-Expressionsvektor pAcSGHisNT-A.

Der Fachmann kennt geeignete Zellen, um die erfindungsgemäße, in einem Expressionsvektor vorliegende DNA zu exprimieren. Beispiele solcher Zellen umfassen die E.coli-Stämme HB101, DH1, x1776, JM101, JM 109, BL21, SG 13009 und M15pRep4, den Hefe-Stamm Saccharomyces cerevisiae, die tierischen Zellen L, NIH 3T3, FM3A, CHO, COS, Vero, HeLa, Hep62, CCL13 und 293, die Insektenzellen Sf9 und Sf21 und die Pflanzenzellen Lupinus albus.

Der Fachmann kennt Verfahren und Bedingungen Zellen mit einem die erfindungsgemäße DNA enthaltenden Expressionsvektor zu transformieren bzw. transfizieren und die Zellen zu kultivieren. Auch sind ihm Verfahren bekannt, das durch die erfindungsgemäße DNA exprimierte Virus-Protein zu isolieren und zu reinigen.

Ein gegen ein vorstehendes Fusionsprotein gerichteter Antikörper kann durch übliche Verfahren hergestellt werden. Er kann polyklonal bzw. monoklonal sein. Zu seiner Herstellung ist es günstig, Tiere, insbesondere Kaninchen oder Hühner für einen polyklonalen und Mäuse für einen monoklonalen Antikörper, mit dem Fusionsprotein zu immunisieren. Weitere "Booster" der Tiere können ebenfalls mit dem Fusionsprotein erfolgen. Der polyklonale Antikörper kann dann aus dem Serum bzw. Eigelb der Tiere erhalten werden. Für den monoklonalen Antikörper werden Milzzellen der Tiere mit Myelomzellen fusioniert.
(a) Ein erfindungsgemäßes Fusionsprotein,
(b) eine erfindungsgemäße DNA,
(c) ein Antikörper wie vorstehend beschrieben, sowie
(d) übliche Hilfsstoffe, wie Träger, Puffer, Lösungsmittel, Kontrollen, etc.
können als Komponenten in einem Kit vorliegen.

Von den einzelnen Komponenten können jeweils ein oder mehrere Vertreter vorliegen. Hinsichtlich der einzelnen Ausdrücke wird auf vorstehende Ausführungen verwiesen.

Die vorliegende Erfindung stellt ein Gentransfer-System bereit, das spezifisch ist, d.h. mit dem gewünschte Zellen erreicht und in diese Gene eingeführt werden können. Die Zellen können einzeln oder in einem Gewebe vorliegen. Ferner können die Zellen isoliert oder im Körper eines Individuums vorliegen. Somit eignet sich die vorliegende Erfindung für eine ex vivo bzw. in vivo Gentherapie von Zellen bzw. Geweben. Die Anwendung der vorliegenden Erfindung kann dabei durch erfindungsgemäße Antikörper überwacht und gesteuert werden.

Somit stellt die vorliegende Erfindung einen großen Schritt dar gentherapeutische Veränderungen an Zellen bzw. Geweben gezielt durchzuführen.

### Kurze Beschreibung der Zeichnungen.

- Fig. 1: zeigt die Aminosäure- und DNA-Sequenzen eines erfindungsgemäßen Fusionsproteins, das ein LHBs und die heterologe Bindungsstelle RGD umfaßt, wobei diese die homolge ersetzt.
- Fig. 2: zeigt die Aminosäure- und DNA-Sequenzen eines erfindungsgemäßen Fusionsproteins, das ein HBcAg, ein Zellpermeabilität-vermittelndes Peptid der vorstehenden Aminosäuresequenz und die heterologe Bindungsstelle RGD umfaßt.

Die vorliegende Erfindung wird durch die nachstehenden Beispiele erläutert.

### Beispiel 1: Herstellung eines erfindungsgemäßen Partikels, das ein Fusionsprotein enthält, welches ein LHBs und eine heterologe Bindungsstelle umfaßt.

### (A) Herstellung eines Expressionsvektor, der für sämtliche HBV-spezifischen Proteine mit Ausnahme von LHBs kodiert.

Hierzu wird von dem Plasmid pTKTHBV2 (vgl. Will et al., Proc. Natl. Acad. Sci. 82 (1985), 891-895) ausgegangen. Dieses enthält zwei Kopien des HBV-Genoms. In einer ersten PCR wird ein Fragment von ntHBV2821 (erste Kopie) bis ntHBV2870 (zweite Kopie) amplifiziert. Der forward Primer (nt 2821-2855) weist folgende Sequenz auf: CCA TAT TCT TGG GAA CAA GAT ATC CAG CAC GGG GC. Eine EcoRV-Schnittstelle ist unterstrichen. Das Triplett ACG zwischen nt 2849-2852 ersetzt das ATG-Startcodon von LHBs. Der backward Primer (nt 2877-2845) weist folgende Sequenz auf: GGA TTG CTG GTG GAA GAT ATC TGC CCC GTG CTG. Eine EcoRV-Schnittstelle ist unterstrichen. Das Triplett CGT zwischen nt 2852-2849 ersetzt das natürliche Triplett CAT. Erhaltene PCR-Fragmente werden mit EcoRV verdaut und über ein präparatives 1%iges Agarosegel gereinigt. Ein Fragment der Größe von ca. 3, 3 kb wird aus dem Gel eluiert und aufbewahrt.

In einer zweiten PCR werden ein forward Primer, der eine EcoRV-Schnittstelle gefolgt von der nachstehenden Sequenz ntHBV2860 (zweite Kopie) -2878 (erste Kopie) (CAG CAC GGG GCA GAT ATC TTC CAC CAG CAA TCC) aufweist, und ein backward Primer verwendet, der eine EcoRV Schnittstelle gefolgt von der nachstehenden Sequenz ntHBV 2830-2810 (GC CCC GTG CTG GAT ATC ATC TTG TTC CCA AGA ATA TGG) aufweist. Erhaltende PCR-Fragmente werden mit EcoRV verdaut und über ein präparatives 1 % Agarose-Gel gereinigt. Ein Fragment der erwarteten Größe wird aus dem Gel eluiert und dephosphoryliert. Dieses Fragment wird mit dem vorstehenden ca. 3,3 kb großen in einer Ligasereaktion eingesetzt, wodurch der HBV-Expressionsvektor pTKTHBV2Ldef erhalten wird. Dieser Expressionsvektor kodiert für sämtliche HBV-spezifischen Proteine mit Ausnahme von LHBs.

### (B) Herstellung eines Expressionsvektors, der für ein Fusionsprotein kodiert, welches ein LHBs und die heterologe Bindungsstelle RGD umfaßt.

Ausgehend von dem Plasmid pTKTHBV2 (vgl. vorstehend) wird das Fragment ntHBV2990-834 durch PCR amplifiziert. Der 5'-Primer weist folgende Sequenz auf: AAA AGA TCT GGC CGT GGC GAA GGA GCT GGA GCA TTC. Diese umfaßt eine BglII-Schnittstelle, gefolgt von einem ATG-Startcodon und der für das Tripeptid RGD-kodierenden Sequenz. Der PreS1-spezifische Leserahmen wird genutzt. Der 3'-Primer wiest die folgende Sequenz auf: AAA AGA TCT GGT TTA AAT GTA TAC CCA AAG. Diese umfaßt eine BglII-Schnittstelle. Erhaltene PCR-Fragmente werden mit BglII verdaut und in den mit BglII-gespaltenen und dephosphorylierten Vektor pCDNA.3 (Invitrogen) inseriert, wodurch der Expressionsvektor pCRGDLHBs erhalten wird. Dieser Expressionsvektor kodiert für ein N-terminal verkürztes LHBs, das die RGD-Bindungsstelle umfaßt.

### (C) Herstellung eines ein struktur-Gen und ein Verpackungssignal aufweisenden Expressionsvektors

Es wird eine für das HBV-Verpackungssignal Epsilon Kodiererde Sequenz, z.B. ntHBV 1840-1914, mittels PCR amplifiziert. Durch die verwendeten Primer wird eine EcoRV-Schnittstelle eingeführt. Die Sequenz des forward Primers lautet: CCC GAT ATC ATG TCA TCT CTT GTT CAT GTC CTA. Die Sequenz des backward Primers lautet: GGG GAT ATC GGT CGA TGT CCA TGC CCC AAA. Erhaltene PCR-Fragmente werden mit EcoRV gespalten und in den mit EcoRV gespaltenen und dephosphorylierten Vektor pCDNA.3 (vgl. vorstehend) inseriert, wodurch der Vektor pcVPHBV erhalten wird. Dieser Vektor enthält das HBV-spezifische Verpackungssignal Epsilon.

Ausgehend von dem Vektor pCeGFP (Invitrogen), der für ein "green fluorescent protein" unter der Kontrolle des CMV-Promotors kodiert, wird die den CMV-Promotor und das GFP-Gen enthaltende Sequenz mittels PCR amplifiziert. Der forward Primer hat folgende Sequenz: GGG GGA TCC CGA TGT ACG GGC CAG ATA TAC GCG TTG. Der backward Primer hat folgende Sequenz: GGG GGA TCC GCG GCC GCT TTA CTT GTA. Die verwendeten Primer enthalten jeweils eine BamHI-Schnittstelle. Erhaltene PCR-Fragmente werden mit BamHI gespalten und in den mit BamHI gespaltenen und dephosphorylierten Vektor pCVPHBV (Invitrogen) inseriert, wodurch der Expressionsvektor pCVPHBVeGFP erhalten wird. Dieser Expressionsvektor enthält das HBV-spezifische Verpackungssignal Epsilon, den CMV-Promotor und eine für eGFP kodierende Sequenz.

### (D) Herstellung einer Verpackungszellinie

Etwa 0.8x10⁶ HepG2-Zellen werden mit 4 µg von pTKTHBV2Ldef (vgl. (A)) und 2 µg pCDNA.3 (vgl. (B)) mittels Lipofektion transfiziert. pCDNA.3 kodiert für G418 Resistenz. 2h nach Transfektion werden die Zellen in ein 700 mg G418/l enthaltendes Medium überführt. G418 resistente Klone werden nach 14d subkultiviert. Die stabile Integration von pTKTHBV2Ldef wird mittels PCR und Southern Blots nachgewiesen. Die Expression des Oberflächenproteins SHBs von HBV und von HBcAg wird mittels spezifischer Antikörper in ELISAS nachgewiesen. Es wird die Verpackungszellinie HepG2-TKTHBV2Ldef erhalten. Diese Zellinie exprimiert sämtliche HBV-spezifischen Proteine mit Ausnahme von LHBs.

### (E) Herstellung erfindungsgemäßer Partikel

Etwa 0.8x10⁶ Zellen der Verpackungszellinie von (D) werden mit 3 µg von pCRGDLHBs (vgl. (B)) und 3 µg von pCVPHBVeGFP (vgl. (B)) mittels Lipofektion transfiziert. 72 h nach Transfektion weren die Zellen bzw. deren Überstände gesammelt und einer PEG-Fällung unterworfen. Anschließend wird eine CsCl-Dichtegradienten-Zentrifugation durchgeführt. Es werden erfindungsgemäße Partikel in reiner Form erhalten. Diese Partikel umfassen sämtliche HBV-spezifischen Proteine mit Ausnahme von LHBs, das durch ein RGD-LHBs ersetzt ist.

### Beispiel 2: Herstellung eines erfindungsgemäßen Partikels, das ein Fusionsprotein enthält, welches ein HBcAg, ein Zellpermeabilität-vermittelndes Peptid und eine heterologe Bindungsstelle umfaßt.

Es wird eine für ein Zellpermeabilität-vermittelndes Peptid (nachstehend mit ZPP bezeichnet) kodierende DNA verwendet. Diese hat folgende Sequenz: XXX AGA TCT ATC CCC ATA TCG TCA ATC TTC TCG AGG ATT GGG GAC CCT GGA TCC XXX (X bezeichnet ein beliebiges Nuklectid). Diese weist am 5'-Ende eine BglII-Schnittstelle, gefolgt von einem ATG-Startcodon und an ihrem 3'-Ende eine BamHI-Schnittstelle auf. Ein doppelsträngiges DNA-Molekül, das auf vorstehender Sequenz basiert, wird mit BamHI/BglII geschnitten und in den mit BamHI-gespaltenen und dephosphcrylierten Expressionsvektor pCDNA.3 (vgl. vorstehend) inseriert, wodurch der Expressionsvektor pCZPP erhalten wird.

Ferner wird der Expressionsvektor pTKTHBV2 (vgl. vorstehend) verwendet, um das Fragment nt-HBV 1861-2136 mittels PCR zu amplifizieren. Der forward-Primer umfaßt die folgende Sequenz: XXX GGA TCC ACT GTT CTA GCC TCC AAG CTG. Diese umfaßt eine BamII-Schnittstelle gefolgt von der Sequenz ntHB 1861-1881 Der backward Primer umfaßt die folgende Sequenz: XXX GAA TTC TGG ATC TTC CAA ATT AAC ACC CAC CCA. Diese umfaßt eine EcoRI Schnittstelle gefolgt von der Sequenz ntHBV 2139-2116. In einer zweiten PCR wird das Fragment ntHBV 2140-2480 amplifiziert, das an seinem 5'-Ende mit der für das RGD-Motiv kodierenden Sequenz erweitert ist. Der forward Primer umfaßt die folgende Sequenz: XXX GAA TTC CGA GGC GAC GCG TCT AGA GAC CTA GTA GTC. Diese umfaßt eine EcoRI-Schnittstelle gefolgt von der für das RGD-Motiv kodierenden Sequenz und der Sequenz ntHBV2140-2161. Der backward Primer umfaßt die folgende Sequenz: XXX AAG CTT TCC CCA CCT TAT GAG TCC AAG. Diese umfaßt eine HindIII-Schnittstelle und die Sequenz ntHBV 2480-2460.

Erhaltene Fragmente beider PCRs werden mit EcoRI gespalten und miteinander ligiert. Das Ligationsprodukt wird als Template für eine weitere PCR verwendet, wobei als forward Primer jener der ersten PCR und als backward Primer jener der zweiten PCR verwendet werden. Erhaltene PCR-Fragmente werden mit BamHI/ HindIII gespalten und in den mit BamHI/HindIII-gespaltenen und dephosphorylierten Vektor pCZPP inseriert, wodurch der Expressionsvektor pCZPPHBcRGC erhalten wird. Dieser Expressionsvektor kodiert für HBcAg, das N-terminal die ZPP-Sequenz und im Bereich der Aminosäuren 79-82 die RGD-Sequenz enthält.

Des weiteren werden etwa 0.8x10⁶ HepG2-Zellen mittels Liopfektion mit 4 µg eines für HBV-Polymerase kodierenden Expressionsvektors und mit 2 µg pCDNA.3 transfiziert. Es wird auf die vorstehende Beschreibung von Beispiel 1, (D) verwiesen. Es wird eine mit HepG2-HBV Pol bezeichnete Zellinie erhalten.

Etwa 0.8x10⁶ Zellen der Zellinie HepG2-HBV Pol werden mit 3 µg von pCZPPHBcRGC und 3 µg von pCVPHBVeGFP (vgl. Beispiel 1,B) mittels Lipofektion transfiziert. Es wird auf vorstehende Beschreibung von Beispiel 1, (E) verwiesen. Es werden erfindungsgemäße Partikel in reiner Form erhalten.

### Beispiel 3: Nachweis der Expression einer in erfindungsgemäßen Partikeln vorliegenden Nukleinsäure in Fibroblasten

Etwa 1x10⁹ erfindungsgemäße Partikel von Beispiel 1 (E) bzw. Beispiel 2, werden in 100 µl 0,9 % Saline gelöst und in die Schwanzvene von balb/c Mäusen injiziert. 48 h nach Injektion wird der Soleus- und der Tibialis anterior Muskel isoliert und in einem "tissue tag" langsam eingefroren. Aus den eingefrorenen Präparaten werden Kryoschnitte angefertigt und diese unter einem Fluoreszenzmikroskop bei Blauanregung analysiert.

Es wird eine grüne Fluoreszenz in den Fibroblasten erhalten, was die Expression des "green fluorescent protein" zeigt.

### Beispiel 4: Herstellung und Reinigung eines erfindungsgemäßen Fusionsproteins

Es wird das erfindungsgemäße Fusionsprotein von Fig. 1 hergestellt. Hierzu wird die DNA von Fig. 1 am 5'-Ende mit einem BglII-Linker und am 3'-Ende mit einem BglII-Linker versehen und mit den entsprechenden Restriktionsenzymen nachgespalten. Das erhaltene BglII/BglII-Fragment wird in den BamHI-gespaltenen Expressionsvektor pQE3 inseriert, so daß das Expressionsplasmid pQE8/LHBs erhalten wird. Ein solches kodiert für ein Fusionsprotein aus 6 Histidin-Resten (N-Terminuspartner) und dem erfindungsgemäßen Fusionsprotein von Fig. 1 (C-Terminuspartner). pQE-8/LHBs wird zur Transformation von E.coli SG 13009 (vgl. Gottesman, S. et al. , J. Bacteriol. 148, (1981), 265-273) verwendet. Die Bakterien werden in einem LB-Medium mit 100 µg/ml Ampicillin und 25 µg/ml Kanamycin kultiviert und 4 h mit 60 µM Isopropyl-β-D-Thiogalactopyranosid (IPTG) induziert. Durch Zugabe von 6 M Guanidinhydrochlorid wird eine Lyse der Bakterien erreicht, anschließend wird mit dem Lysat eine Chromatographie (Ni-NTA-Resin) in Gegenwart von 8 M Harnstoff entsprechend der Angaben des Herstellers (Qiagen) des Chromatographie-Materials durchgeführt. Das gebundene Fusionsprotein wird in einem Puffer mit pH 3,5 eluiert. Nach seiner Neutralisierung wird das Fusionsprotein einer 18 % SDS-Polyacrylamid-Gelelektrophorese unterworfen und mit Coomassie-Blau angefärbt (vgl. Thomas, J.O. und Kornberg, R.D., J. Mol. Biol. 149, (1975), 709-733).

Es zeigt sich, daß ein erfindungsgemäßes Fusionsprotein in hochreiner Form hergestellt werden kann.

### Beispiel 5: Herstellung und Nachweis eines Antikörpers

Ein erfindungsgemäßes Fusionsprotein von Beispiel 4 wird einer 18 %igen SDS-Polyacrylamid-Gelelektrophorese unterzogen. Nach Anfärbung des Gels mit 4 M Natriumacetat wird eine 38 kD Bande aus dem Gel herausgeschnitten und in Phosphat gepufferter Kochsalzlösung inkubiert. Gel-Stücke werden sedimentiert, bevor die Proteinkonzentration des Überstandes durch eine SDS-Polyacrylamid-Gelelektrophorese, der eine Coomassie-Blau-Färbung folgt, bestimmt wird. Mit dem Gel-gereinigten Fusionspolypeptid werden Tiere wie folgt immunisiert:

### Immunisierungsprotokoll für polyklonale Antikörper im Kaninchen

Pro Immunisierung werden 35 µg Gel-gereinigtes Fusionsprotein in 0,7 ml PBS und 0,7 ml komplettem bzw, inkomplettem Freund's Adjuvans eingesetzt.

| | |
|---|---|
| Tag O: | 1. Immunisierung (komplettes Freund's Adjuvans) |
| Tag 14: | 2. Immunisierung (inkomplettes Freund's Adjuvans; icFA) |
| Tag 28: | 3. Immunisierung (icFA) |
| Tag 56: | 4. Immunisierung (icFA) |
| Tag 80: | Ausbluten |

Das Serum des Kaninchens wird im Immunoblot getestet. Hierzu wird ein erfindungsgemäßes Fusionsprotein von Beispiel 4 einer SDS-Polyacrylamid-Gelelektrophorese unterzogen und auf ein Nitrocellulosefilter übertragen (vgl. Khyse-Andersen, J., J. Biochem. Biophys. Meth. 10, (1984), 203-209). Die Western Blot-Analyse wurde wie in Bock, C.-T. et al., Virus Genes 8, (1994), 215-229, beschrieben, durchgeführt. Hierzu wird das Nitrocellulosefilter eine Stunde bei 37°C mit einem ersten Antikörper inkubiert. Dieser Antikörper ist das Serum des Kaninchens (1:10000 in PBS). Nach mehreren Waschschritten mit PBS wird das Nitrocellulosefilter mit einem zweiten Antikörper inkubiert. Dieser Antikörper ist ein mit alkalischer Phosphatase gekoppelter monoklonaler Ziege Anti-Kaninchen-IgG-Antikörper (Dianova) (1:5000) in PBS. Nach 30-minütiger Inkubation bei 37°C folgen mehrere Waschschritte mit PBS und anschließend die alkalische Phosphatase-Nachweisreaktion mit Entwicklerlösung (36 µM 5' Bromo-4-chloro-3-indolylphosphat, 400 µM Nitroblau-tetrazolium, 100 mM Tris-HCl, pH 9.5, 100 mM NaCl, 5 mM MgCl₂) bei Raumtemperatur, bis Banden sichtbar werden.

Es zeigt sich, daß gegen ein erfindungsgemäßes Fusionsprotein gerichtete, polyklonale Antikörper hergestellt werden können.

### Immunisierungsprotokoll für polyklonale Antikörper im Huhn

Pro Immunisierung werden 40 µg Gel-gereinigtes Fusionsprotein in 0,8 ml PBS und 0,8 ml komplettem bzw. inkomplettem Freund's Adjuvans eingesetzt.

| | |
|---|---|
| Tag O: | 1. Immunisierung (komplettes Freund's Adjuvans) |
| Tag 28: | 2. Immunisierung (inkomplettes Freund's Adjuvans; icFA) |
| Tag 50: | 3. Immunisierung (icFA) |

Aus Eigelb werden Antikörper extrahiert und im Western Blot getestet. Es werden, gegen ein erfindungsgemäßes Fusionsprotein gerichtete polyklonale Antikörper nachgewiesen.

### Immunisierungsprotokoll für monoklonale Antikörper der Maus

Pro Immunisierung werden 12 µg Gel-gereinigtes Fusionsprotein in 0,25 ml PBS und 0,25 ml komplettem bzw. inkomplettem Freund's Adjuvans eingesetzt; bei der 4. Immunisierung ist das Fusionsprotein in 0,5 ml (ohne Adjuvans) gelöst.

| | |
|---|---|
| Tag O: | 1. Immunisierung (komplettes Freund's Adjuvans) |
| Tag 28: | 2. Immunisierung (inkomplettes Freund's Adjuvans; icFA) |
| Tag 56: | 3. Immunisierung (icFA) |
| Tag 84: | 4. Immunisierung (PBS) |
| Tag 87: | Fusion |

Überstände von Hybridomen werden im Western Blot getestet. Gegen ein erfindungsgemäßes Fusionsprotein gerichtete monoklonale Antikörper werden nachgewiesen.

## Patentansprüche

1. Partikel, umfassend:
(a) eine Proteinhülle mit einem Fusionsprotein, das ein Virus-Protein, ein Zellpermeabilität-vermittelndes Peptid und eine heterologe zellspezifische Bindungsstelle umfasst, und
(b) eine in der Proteinhülle vorliegende Nukleinsäure, die Sequenzen für ein Virus-spezifisches Verpackungssignal und ein Struktur-Gen aufweist,
wobei das Zellpermeabilität-vermittelnde Peptid die Aminosäuresequenz PLSSIFSRIGDP oder eine Aminosäuresequenz, die zu der Aminosäuresequenz P L S S I F S R I G D P in einer Aminosäure unterschiedlich ist, umfasst, wobei die Aminosäuresequenz, die zu der Aminosäuresequenz P L S S I F S R I G D P in einer Aminosäure unterschiedlich ist, von einer Nukleinsäuresequenz kodiert wird, die mit der Nukleinsäuresequenz CCC CTA TCG TCA ATC TTC TCG AGG ATT GGG GAC CCT hybridisiert.

2. Partikel nach Anspruch 1, wobei das Virus-Protein von einem Adenovirus, Adeno-assoziierten Virus, Vacciniavirus, Baculovirus oder Hepadna-Virus stammt.

3. Partikel nach Anspruch 2, wobei das Hepadna-Virus ein Hepatitis B-Virus ist.

4. Partikel nach einem der Ansprüche 1-3, wobei das Virus-Protein ein Oberflächenprotein ist.

5. Partikel nach Anspruch 4, wobei das Oberflächenprotein ein LHBs ist.

6. Partikel nach einem der Ansprüche 1-3, wobei das Virus-Protein ein Core-Protein ist.

7. Partikel nach Anspruch 6, wobei das Core-Protein ein HBcAg ist.

8. Partikel nach einem der Ansprüche 1-7, wobei das Zellpermeabilität-vermittelnde Peptid die folgende Aminosäuresequenz aufweist: PLSSIFSRIGDP.

9. Partikel nach einem der Ansprüche 1-8, wobei die heterologe zellspezifische Bindungsstelle RGD ist.

10. Partikel nach einem der Ansprüche 1-9, wobei das Fusionsprotein jenes von Fig. 1 oder 2 ist.

11. Verfahren zur Herstellung des Partikels nach Anspruch 1, wobei das Fusionsprotein ein LHBs und eine heterologe zellspezifische Bindungsstelle enthält, umfassend die folgenden Verfahrensschritte:
(a) Co-Transfektion von Zellen, die für ein Heptatitis B-Virus-Genom kodieren, wobei diese kein LHBs exprimieren, mit einem ersten Expressionsvektor, der für ein Fusionsprotein kodiert, das ein LHBs und eine heterologe zellspezifische Bindungsstelle umfasst, und mit einem zweiten Expressionsvektor, der ein Virus-spezifisches Verpackungssignal und ein Struktur-Gen aufweist, und
(b) Isolierung und Reinigung des Partikels.

12. Verfahren zur Herstellung des Partikels nach Anspruch 1, wobei das Fusionsprotein ein HBcAg, ein Zellpermeabilität-vermittelndes Peptid und eine heterologe zellspezifische Bindungsstelle aufweist, umfassend die folgenden Verfahrensschritte:
(a) Co-Transfektion von Zellen, die für eine HBV-Polymerase kodieren, mit einem ersten Expressionsvektor, der für ein Fusionsprotein kodiert, das ein HBcAg, ein Zellpermeabilität-vermittelndes Peptid und eine heterologe zellspezifische Bindungsstelle umfasst, und mit einem zweiten Expressionsvektor, der ein Virus-spezifisches Verpackungssignal und ein Struktur-Gen aufweist, und
(b) Isolierung und Reinigung des Partikels.

13. Fusionsprotein, umfassend ein Virus-Protein, ein Zellpermeabilität-vermittelndes Peptid und eine heterologe zellspezifische Bindungsstelle, wobei das Zellpermeabilität-vermittelnde Peptid die Aminosäuresequenz P L S S I F S R I G D P oder eine Aminosäuresequenz, die zu der Aminosäuresequenz P L S S I F S R I G D P in einer Aminosäure unterschiedlich ist, umfasst, wobei die Aminosäuresequenz, die zu der Aminosäuresequenz P L S S I F S R I G D P in einer Aminosäure unterschiedlich ist, von einer Nukleinsäuresequenz kodiert wird, die mit der Nukleinsäuresequenz CCC CTA TCG TCA ATC TTC TCG AGG ATT GGG GAC CCT hybridisiert.

14. Fusionsprotein nach Anspruch 13, umfassend die Aminosäuresequenz von Fig. 1 oder 2 oder eine hiervon durch eine oder mehrere Aminosäuren unterschiedliche Aminosäuresequenz, die sich von der Aminosäuresequenz von Fig. 1 oder 2 um bis zu 20% unterscheidet.

15. DNA, kodierend für das Fusionsprotein nach Anspruch 13.

16. DNA, kodierend für das Fusionsprotein nach Anspruch 14, umfassend:
(a) die DNA von Fig. 1 oder 2 oder eine hiervon durch ein oder mehrere Basenpaare unterschiedliche DNA, oder
(b) eine mit der DNA von (a) über den degenerierten gentischen Code verwandte DNA.

17. Expressionsvektor, kodierend für die DNA nach Anspruch 16.

18. Verwendung des Partikels nach einem der Ansprüche 1-10 zur Herstellung einer pharmazeutischen Zusammensetzung zur Gentherapie von Zellen und Geweben.

## Claims

1. A particle comprising:
(a) a protein envelope with a fusion protein, the fusion protein comprising a virus protein, a cell permeability-mediating peptide, and a heterologous cell-specific binding site; and
(b) a nucleic acid present within the protein envelope comprising sequences for a virus-specific packaging signal and a structural gene,
wherein the cell permeability-mediating peptide comprises the amino acid sequence PLSSIFSRIGDP or an amino acid sequence differing from the amino acid sequence PLSSIFSRIGDP in one amino acid, wherein the amino acid sequence differing from the amino acid sequence PLSSIFSRIGDP in one amino acid is encoded by a nucleic acid sequence hybridizing with the nucleic acid sequence CCC CTA TCG TCA ATC TTC TCG AGG ATT GGG GAC CCT.

2. The particle of claim 1, wherein the virus protein is derived from an adenovirus, adeno-associated virus, vaccinia virus, baculovirus or hepadna virus.

3. The particle of claim 2, wherein the hepadna virus is a hepatitis B virus.

4. The particle of any one of claims 1-3, wherein the virus protein is a surface protein.

5. The particle of claim 4, wherein the surface protein is an LHBs.

6. The particle of any one of claims 1-3, wherein the virus protein is a core protein.

7. The particle of claim 6, wherein the core protein is an HBcAg.

8. The particle of any one of claims 1-7, wherein the cell permeability-mediating peptide has the following amino acid sequence: PLSSIFSRIGDP.

9. The particle of any one of claims 1-8, wherein the heterologous cell-specific binding site is RGD.

10. The particle of any one of claims 1-9, wherein the fusion protein is that of fig. 1 or 2.

11. A method for the preparation of the particle of claim 1, wherein the fusion protein contains an LHBs and a heterologous cell-specific binding site, the method comprising the following steps:
(a) co-transfecting cells coding for a hepatitis B virus genome, wherein the cells do not express LHBs, with a first expression vector coding for a fusion protein comprising an LHBs and a heterologous cell-specific binding site, and with a second expression vector comprising a virus-specific packaging signal and a structural gene; and
(b) isolating and purifying the particle.

12. A method for the preparation of the particle of claim 1, wherein the fusion protein comprises an HBcAg, a cell permeability-mediating peptide and a heterologous cell-specific binding site, the method comprising the following steps:
(a) co-transfecting cells coding for an HBV polymerase with a first expression vector coding for a fusion protein comprising an HBcAg, a cell permeability-mediating peptide and a heterologous cell-specific binding site, and with a second expression vector comprising a virus-specific packaging signal and a structural gene; and
(b) isolating and purifying the particle.

13. A fusion protein comprising a virus protein, a cell permeability-mediating peptide, and a heterologous cell-specific binding site, wherein the cell permeability-mediating peptide comprises the amino acid sequence PLSSIFSRIGDP or an amino acid sequence differing from the amino acid sequence PLSSIFSRIGDP in one amino acid, wherein the amino acid sequence differing from the amino acid sequence PLSSIFSRIGDP in one amino acid is encoded by a nucleic acid sequence hybridizing with the nucleic acid sequence CCC CTA TCG TCA ATC TTC TCG AGG ATT GGG GAC CCT.

14. The fusion protein of claim 13, comprising the amino acid sequence set forth in fig. 1 or 2 or an amino acid sequence differing therefrom in one or more amino acids and differing from the amino acid sequence set forth in fig. 1 or 2 in up to 20%.

15. A DNA coding for the fusion protein of claim 13.

16. A DNA coding for the fusion protein of claim 14, the DNA comprising:
(a) the DNA set forth in fig. 1 or 2 or a DNA differing therefrom in one or more base pairs; or
(b) a DNA being related to the DNA under (a) via the degenerated genetic code.

17. An expression vector coding for the DNA of claim 16.

18. A use of the particle of any one of claims 1-10 for preparing a pharmaceutical composition for gene therapy of cells and tissues.

## Revendications

1. Particule, comprenant :
(a) une enveloppe protéique avec une protéine de fusion, qui comprend une protéine virale, un peptide conférant une perméabilité cellulaire et une position de liaison hétérologue, spécifique pour une cellule, et
(b) un acide nucléique présent dans l'enveloppe protéique, qui présente des séquences pour un signal de conditionnement spécifique d'un virus et un gène de structure,
tandis que le peptide conférant la perméabilité cellulaire présente la séquence d'acides aminés PLSSIFSRIGDP ou une séquence d'acides aminés qui diffère de la séquence d'acides aminés PLSSIF SRIGDP par un acide aminé, tandis que la séquence d'acides aminés qui diffère de la séquence d'acides aminés PLSSIFSRIGD P par un acide aminé est codée par une séquence d'acide nucléique qui hybride avec la séquence d'acide nucléique CCC CTA TCG TCA ATC TTC TCG AGG ATT GGG GAC CCT.

2. Particule selon la revendication 1, dans laquelle la protéine virale provient d'un adénovirus, d'un virus adéno-associé, d'un virus de la vaccine, d'un baculovirus ou d'un hepadnavirus.

3. Particule selon la revendication 2, dans laquelle l'hepadnavirus est un virus de l'hépatite B.

4. Particule selon l'une des revendications 1-3, dans laquelle la protéine virale est une protéine de surface.

5. Particule selon la revendication 4, dans laquelle la protéine de surface est un LHBs.

6. Particule selon l'une des revendications 1-3, dans laquelle la protéine virale est une protéine de noyau.

7. Particule selon la revendication 6, dans laquelle la protéine de noyau est un HBcAg.

8. Particule selon l'une des revendications 1-7, dans laquelle le peptide conférant une perméabilité cellulaire présente la séquence d'acides aminés suivante : PLSSIFSRIGDP.

9. Particule selon l'une des revendications 1-8, dans laquelle la position de liaison hétérologue spécifique pour la cellule est RGD.

10. Particule selon l'une des revendications 1-9, dans laquelle la protéine de fusion est l'une quelconque de celles de Fig. 1 ou 2.

11. Procédé pour la préparation de la particule selon la revendication 1, dans lequel la protéine de fusion contient un LHBs et une position de liaison hétérologue, spécifique pour une cellule, comportant les étapes opératoires suivantes :
(a) co-transfection de cellules qui codent pour un génome du virus de l'hépatite B, tandis que celles-ci n'expriment pas de LHBs, avec un premier vecteur d'expression qui code pour une protéine de fusion qui comporte un LHBs et une position de liaison hétérologue, spécifique pour une cellule, et avec un deuxième vecteur d'expression qui présente un signal de conditionnement spécifique pour un virus et un gène de structure, et
(b) isolement et purification de la particule.

12. Procédé pour la préparation de la particule selon la revendication 1, dans lequel la protéine de fusion présente un HBcAg, un peptide conférant une perméabilité cellulaire et une position de liaison hétérologue, spécifique pour une cellule, comprenant les étapes opératoires suivantes :
(a) co-transfection de cellules qui codent pour une VHB-polymérase, avec un premier vecteur d'expression qui code pour une protéine de fusion qui comporte un HBcAg, un peptide conférant une perméabilité cellulaire et une position de liaison hétérologue, spécifique pour une cellule, et avec un deuxième vecteur d'expression qui présente un signal de conditionnement spécifique pour un virus et un gène de structure, et
(b) isolement et purification de la particule.

13. Protéine de fusion, comprenant une protéine virale, un peptide conférant une perméabilité cellulaire et une position de liaison hétérologue, spécifique pour une cellule, tandis que le peptide conférant une perméabilité cellulaire présente la séquence d'acides aminés PLSSIFSRIGDP ou une séquence d'acides aminés qui diffère de la séquence d'acides aminés PLSSIFSRIGDP par un acide aminé, tandis que la séquence d'acides aminés qui diffère de la séquence d'acides aminés PLSSIFSRIGDP d'un acide aminé est codée par une séquence d'acide nucléique qui hybride avec la séquence d'acide nucléique CCC CTA TCG TCA ATC TTC TCG AGG ATT GGG GAC CCT.

14. Protéine de fusion selon la revendication 13, comprenant la séquence d'acides aminés selon Fig. 1 ou 2 ou une séquence d'acides aminés qui en diffère par un ou plusieurs acides aminés, et qui se distingue de la séquence d'acides aminés selon Fig. 1 ou 2 d'au plus 20%.

15. ADN codant pour la protéine de fusion selon la revendication 13.

16. ADN codant pour la protéine de fusion selon la revendication 14, comprenant:
(a) l'ADN selon Fig. 1 ou 2 ou un ADN s'en distinguant par une ou plusieurs paires de bases, ou
(b) un ADN apparenté à l'ADN selon (a) par l'intermédiaire du code génétique dégénéré.

17. Vecteur d'expression codant pour l'ADN selon la revendication 16.

18. Utilisation de la particule selon l'une des revendications 1-10 pour la préparation d'une composition pharmaceutique pour la thérapie génique de cellules et de tissus.
